# EUROPEAN PATENT SPECIFICATION

(11) **EP 2 102 662 B1**
(45) Date of publication and mention of the grant of the patent: **15.05.2013**
(21) Application number: 08701443.7
(22) Date of filing: 11.01.2008
(51) Int. Cl.: G01N 33/68

(54) **BIOMARKER FOR THE MEDICINE AND THE BIOLOGY OF THE REPRODUCTION**
BIOMARKER FÜR DIE MEDIZIN UND BIOLOGIE DER REPRODUKTION
BIOMARQUEUR POUR LA MÉDECINE ET LA BIOLOGIE DE LA REPRODUCTION

(30) Priority: 11.01.2007 EP 07300720
(43) Date of publication of application: 23.09.2009
(73) Proprietor: Université d'Aix-Marseille, 13284 Marseille Cedex 07 (FR); INSERM (Institut National de la Santé et de la Recherche Médicale), 75654 Paris Cedex 13 (FR); UNIVERSITE PARIS DESCARTES, 75270 Paris Cedex 06 (FR); Assistance Publique des Hôpitaux de Marseille, 13354 Marseille (FR)
(72) Inventor: PAUL, Pascale, F-13007 Marseille (FR); CAILLAT ZUCMAN, Sophie, F-75014 Paris (FR); PORCU, Géraldine, F-13240 Septèmes-les-Vallons (FR)
(74) Representative: Gallois, Valérie
(86) International application number: PCT/EP2008/050304
(87) International publication number: WO 2008/084105

(56) References cited:
- MINCHEVA-NILSSON LUCIA ET AL: "Placenta-derived soluble MHC class I, chain-related molecules down-regulate NKG2D receptor on peripheral blood mononuclear cells during human pregnancy: A possible novel immune escape mechanism for fetal survival" JOURNAL OF IMMUNOLOGY, vol. 176, no. 6, March 2006 (2006-03), pages 3585-3592, XP002434558 ISSN: 0022-1767 cited in the application
- MINCHEVA-NILSSON L: "Immune cells and molecules in pregnancy: Friends or foes to the fetus?" EXPERT REVIEW OF CLINICAL IMMUNOLOGY 2006 UNITED KINGDOM, vol. 2, no. 3, 2006, pages 457-470, XP001536331 ISSN: 1744-666X
- YIE S-M ET AL: "Secretion of human leukocyte antigen-G by human embryos is associated with a higher in vitro fertilization pregnancy rate" FERTILITY AND STERILITY, ELSEVIER SCIENCE INC, NEW YORK, NY, US, vol. 83, no. 1, January 2005 (2005-01), pages 30-36, XP004713471 ISSN: 0015-0282
- HVIID THOMAS VAUVERT F: "HLA-G in human reproduction: aspects of genetics, function and pregnancy complications" HUMAN REPRODUCTION UPDATE, vol. 12, no. 3, May 2006 (2006-05), pages 209-232, XP002434559 ISSN: 1355-4786
- COULAM ET AL: "Soluble HLA G Concentrations in Culture Media From In Vitro Fertilized Human Embryos are Predicative of Implantation Potential" FERTILITY AND STERILITY, ELSEVIER SCIENCE INC, NEW YORK, NY, US, vol. 84, September 2005 (2005-09), pages S447-S448, XP005103631 ISSN: 0015-0282
- YIE ET AL: "Soluble HLA-G Secreted by Cultured Embryos: A Potential Biomarker for Selection of Embryos for IVF Transfer" FERTILITY AND STERILITY, ELSEVIER SCIENCE INC, NEW YORK, NY, US, vol. 84, September 2005 (2005-09), page S142, XP005102817 ISSN: 0015-0282
- MENIER CATHERINE ET AL: "MICA triggering signal for NK cell tumor lysis is counteracted by HLA-G1-mediated inhibitory signal" INTERNATIONAL JOURNAL OF CANCER, vol. 100, no. 1, 1 July 2002 (2002-07-01), pages 63-70, XP002434560 ISSN: 0020-7136
- MIZUTANI ET AL: "Frequency of MIC Antibody in Rejected Renal Transplant Patients without HLA Antibody" HUMAN IMMUNOLOGY, NEW YORK, NY, US, vol. 67, no. 3, March 2006 (2006-03), pages 223-229, XP005429852 ISSN: 0198-8859
- ZWIRNER NORBERTO W ET AL: "Identification of MICA as a new polymorphic alloantigen recognized by antibodies in sera of organ transplant recipients" HUMAN IMMUNOLOGY, vol. 61, no. 9, September 2000 (2000-09), pages 917-924, XP002473457 ISSN: 0198-8859
- PORCU-BUISSON GERALDINE ET AL: "Soluble MHC Class I chain-related molecule serum levels are predictive markers of implantation failure and successful term pregnancies following IVF" HUMAN REPRODUCTION (OXFORD), vol. 22, no. 8, August 2007 (2007-08), pages 2261-2266, XP002473458 ISSN: 0268-1161
- APPS RICHARD ET AL: "Natural-killer cell ligands at the maternal-fetal interface: UL-16 binding proteins, MHC class-I chain related molecules, HLA-F and CD48", HUMAN REPRODUCTION (OXFORD), vol. 23, no. 11, November 2008 (2008-11), pages 2535-2548, ISSN: 0268-1161

## Description

### Field of the Invention

The present invention relates to the medicine and the biology of the reproduction. The present invention relates to a predictive marker of implantation failure and successful term pregnancies, in particular following *in vitro* fertilization.

### Background of the Invention

Diagnosis and management of infertility is a prevalent health concern in young adults. Ongoing progresses of *in vitro* fertilization (IVF) techniques are challenging reproductive alternatives for infertile couples. *In vitro* fertilization is nevertheless associated with increased risks of clinical complications that represent drawbacks to their indication in women, including risks of multiple births, ectopic pregnancy spontaneous abortion, and preterm delivery. Considering these clinical risks, and the high economic cost of these advanced technique, IVF treatment is still difficult to access for most infertile couples. Although better comprehension of mechanisms that influence fertilization and implantation has provided a more accurate view of parameters associated to *in vitro* fertilization success, search for biomarkers that may predict pregnancy issues after IVF, before initiation of treatment, remains a major challenge. Most of the markers that predict embryo implantation or miscarriage (Tong *et al.,* 2004) occur after initiation of IVF treatment, thus limiting counselling anticipate stress and clinical problems associated with pregnancy failure. Prediction of the chances for infertile women to give birth to a viable baby after IVF treatment is thus a major issue in the optimisation of the medical response to increasing demands of infertile couples.

Therefore, there is a strong need for markers that may improve counselling of infertile couples, as predictors of miscarriage and implantation failure rates associated with IVF at a stage that precedes decision to initiate IVF treatment and engagement of associated clinical complications.

The success of an embryonic implantation passes by the transfer of embryo holding sufficient qualities to be implanted. The capacity of the embryo is appreciated primarily on embryonic morphology and the kinetics of division. The culture of embryo until the stage of blastocystes has several potential advantages among which the selection of the most viable embryos and having the best chances to be implanted, synchronization embryo-uterus, the possibility of evaluating the capacities of the embryonic development in case of multiple implantation failures. One can hope when the transfer was carried out with embryos of good quality (quality defined on the morphological aspect and the speed of development), that the latter will be implanted and allow a pregnancy. The evaluation of the intrinsic capacity of the embryo to emit a set of signals supporting its implantation within the endometrium is still difficult to evaluate.

Therefore, there is a strong need for markers that can help to assess the quality of the embryo, its viability and its capacity to be implanted.

Yie et al (2005, Fertility and Sterility, 83, 30-36) discloses methods and means using soluble HLA-G secreted by human embryos in a culture medium as a marker of the quality of the embryos to be transferred.

Mincheva-Nilsson et al (2006, J. Immunol., 176, 3585-3592) discloses that soluble MICA/B proteins are present during pregnancy and interact with their cognate receptor NKG2D on natural killer cells and may play a immune regulatory roleby silencing the maternal immune system.

Menier et al (2002, International Journal of Cancer, 100, 63-70) discloses that, in their membrane forms, MICA may counteract HLA-G for NK cell.

### Summary of the Invention

The scope of the invention is defined by the claims and any information that does not fall within the claims is provided for information only.

The present invention provides for the first time a biomarker allowing the non-invasive evaluation and prediction of IVF outcome. Besides its prognosis value, a major value of this biomarker is the possibility of its dosage prior initiation of women hormonal conditioning treatment. The use of this biomarker will improve counselling or management of IVF associated-risks and thus provide consequent benefits for infertile women health care. In addition, the inventors identified MICA (MHC class I chain-related protein A) as a marker of infertility and pregnancy complications. Therefore, the present invention concerns MICA as biomarker in the medicine and biology of the reproduction.

In a first embodiment, the present invention concerns a method for determining *in vitro* fertilization (IVF) outcome comprising *in vitro* assaying for soluble MICA in a blood, plasma or serum sample from a woman who receives the transferred embryo, the level of soluble MICA being indicative of the IVF outcome, in particular indicative of implantation failure rates, IVF failure, and/or miscarriage.

In a second embodiment, the present invention concerns a method for selecting a subject for a IVF comprising *in vitro* assaying for soluble MICA in a blood, plasma or serum sample from a woman who is intended to receive the transferred embryo, and selecting the subject having a level of MICA indicative of a successful IVF probability.

In another embodiment, the present invention concerns a method for determining the probability of pregnancy complications, including miscarriage, vascular pregnancy diseases (VPD), preeclampsia (PE), intra-uterine growth retardation (IUGR), associated or not with preeclampsia, HELLP syndrome, gravidic steatosis, gravidic nephropathy or intra-uterine foetal death, pregnancy diseases or infertility associated with auto-immune pathologies in a subject comprising *in vitro* assaying for soluble MICA in a blood, plasma or serum sample, the level of soluble MICA being indicative of a probability of pregnancy complications.

In an aspect of the disclosure, the step of assaying for MICA in the sample comprises a step selected from :
- assaying for soluble MICA protein in the sample;
- assaying for cell-free nucleic acid encoding MICA; and
- assaying for anti-MICA antibodies present in the sample.

In a most preferred embodiment, the step of assaying for MICA in the sample comprises contacting the sample with an anti-MICA antibody, preferably a monoclonal antibody, more preferably selected from the group consisting of SR99, SR104 and SR116. Optionally, the step of assaying for soluble MICA in the sample can be performed by ELISA assay, preferably by sandwich ELISA assay.

In another embodiment, the present invention concerns the *in vitro* use of a kit comprising at least one element selected from the group consisting of an anti-MICA antibody in the medicine and biology of the reproduction for selecting a subject suitable for a IVF, for determining *in vitro* fertilization (IVF) outcome, or for identifying a subject having a high complication risk during pregnancy according to the invention, in particular a subject susceptible to miscarriage, vascular pregnancy diseases, preeclampsia, intra-uterine growth retardation (IUGR), associated or not with preeclampsia, HELLP syndrome, gravidic steatosis, gravidic nephropathy or intra-uterine foetal death.

Preferably, the anti-MICA antibody is a monoclonal antibody, preferably selected from the group consisting of SR99, SR104 and SR116. Optionally, the kit can further comprise a soluble MICA protein.

### Description of the Figures

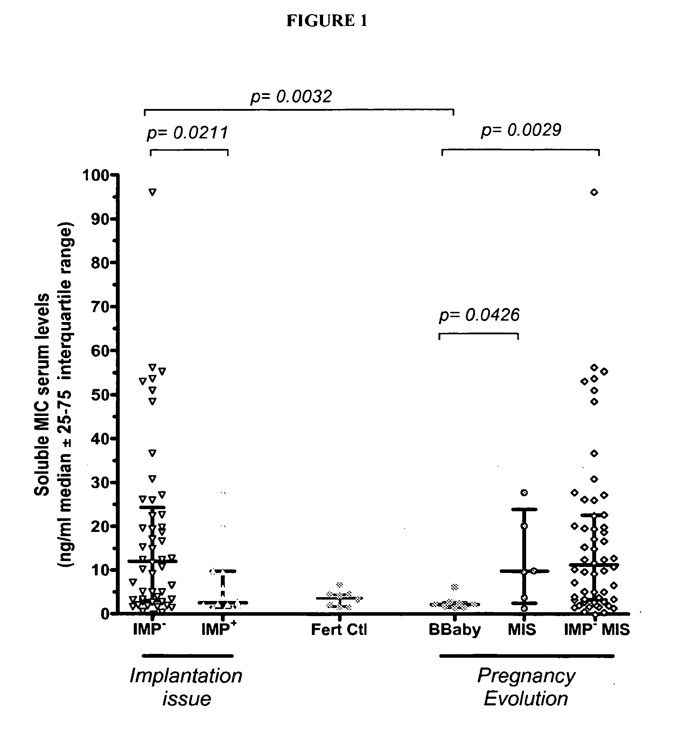

**Figure 1** **: Mean sMIC serum levels in patients differ according to implantation success and IVF issue.** sMIC serum level evaluation was obtained during the follicular phase of the cycle preceding IVF initiation in the serum of 170 infertile women candidate to IVF. sMIC are levels are in ng/ml. Dot plots represent median values in sMIC positive blood samples and error bars 25-75 interquartile ranges of median. Graphics were obtained using GraphPad Prism Version 4 Software. Groups are represented as women that fail to implant after IVF (IMP-), women that experience successful implantation after IVF (IMP+), The IMP+ group is further subdivided in 2 groups: women that give birth to a born baby after IVF (BBaby) or women that experience miscarriage after successful implantation (MIS). Median sMIC values in women that fail to give birth (either because of implantation failure or miscarriage) are also represented as IMP- or MIS. A group of fertile women control donors, not pregnant at blood sample collection, that had experienced at least 2 previous successful pregnancies were also evaluated (Fert Ctl) for s MIC.

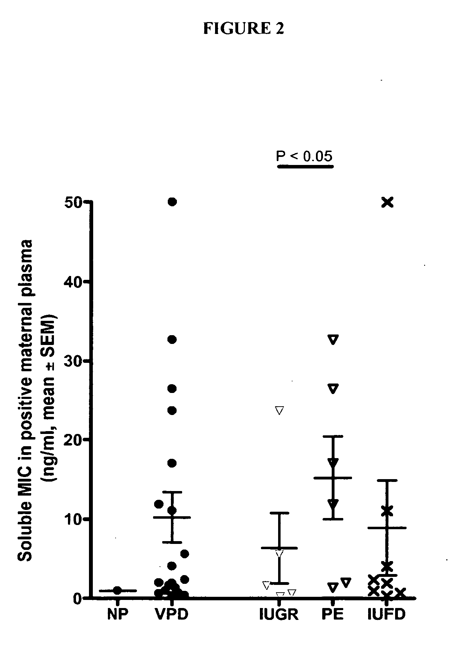

**Figure 2** **:** Higher levels of MIC are found in MIC+ positive samples of women with vascular pregnancy diseases (VPD) in reference with women that undergo normal pregnancy and matched for term (NP). Vascular pregnancy diseases were further subdivided in, intra-uterine growth retardation (IUGR), preeclampsia (PE) and intra-uterine foetal death (IUFD).

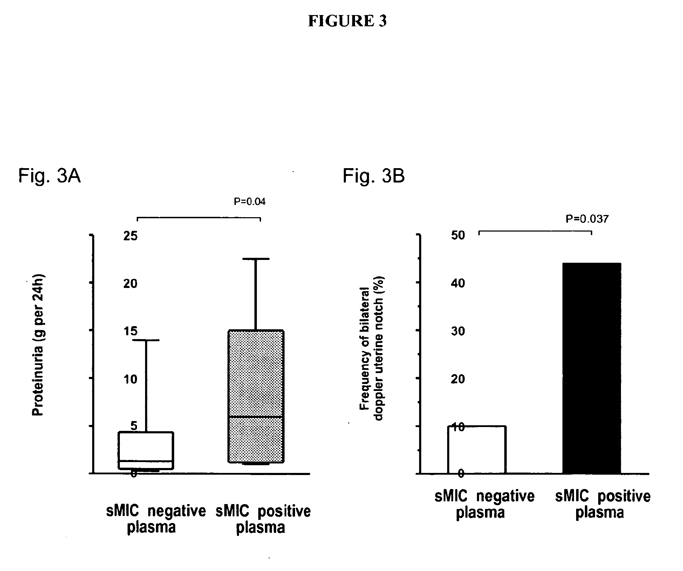

**Figure 3 :** (Fig. 3A) Median and interquartile range of proteinuria per day in preeclampsia group dependent of sMIC status. Comparison between sMIC positive and sMIC negative plasma was performed using non-parametric Mann-withney test. (Fig. 3B) Frequency of bilateral early diastolic uterine notch, in preeclampsia sMIC positive group and sMIC negative plasma from preeclamptic patients.

### Detailed Description of the Invention

The present invention is based on the identification of a biomarker in the field of the medicine and biology of the reproduction. Indeed, they found that the MICA level could be used to predict the IVF outcome, in particular to determine the probability of IVF success or failure, embryo implantation success or failure and miscarriage. In addition, this marker is useful to assess the fertilizing capacity of the semen, and to select the embryos which are the most viable and have the best probability of implantation. This marker is also associated to pregnancy complications. In particular, it can be used for defining the probability of intra-uterine growth retardation (IUGR), intra-uterine foetal death, pregnancy diseases, infertility associated with auto immune pathologies, gravidic vascular diseases and preeclampsia.

MICA has been extensively described in patent applications WO 98/19167 and WO 03/089616, the disclosure of which is incorporated herein by reference. Unigene Cluster for MICA is Hs.549053 and representative sequence can be in Embl Q9UDZ9. Soluble MICA is a truncated protein. Soluble MICA lacks the transmembrane domain and cytoplasmic tail and includes the three extra-cellular domains.

Accordingly, the present disclosure relates to methods for determining *in vitro* fertilization (IVF) outcome comprising *in vitro* assaying for MICA in a sample, the level of MICA being indicative of the IVF outcome. The method can comprise a previous step of providing a sample.

The IVF outcome can be a baby birth, an implantation failure or a miscarriage.

In a preferred embodiment, the method comprises assaying for soluble MICA protein in the sample. In another aspect of the disclosure, the method comprises assaying for cell-free nucleic acid encoding MICA. By "cell-free" is intended the nucleic acid which is not contained into a cell. In an additional aspect of the disclosure, the method comprises an indirect MICA assaying and comprises assaying for anti-MICA antibodies present in the sample.

The sample can be provided from a subject.

In a first embodiment, the subject is the woman who receives the transferred embryo. The subject can also be the oocytes provider. In a particular aspect of disclosure, the sample is selected from the group consisting of a sample of blood, plasma, serum, endometrium biopsy, uterine fluid, vaginal and cervical secretions, cervical mucus, Douglas' pouch, and peritoneal fluid in case of ceolioscopic exploration (endometriosis, adherent pelvis, infectious sequela). In a preferred embodiment, the sample is a sample of blood, plasma, or serum, more preferably a serum sample.

Preferably, the level of soluble MICA is determined prior initiation of hormonal conditioning treatment. In a first particular embodiment, prior initiation of hormonal conditioning treatment, a level of MICA greater than 2.45 ng/ml in the serum sample is indicative of higher implantation failure rates. Accordingly, the present invention concerns a method wherein the subject is the woman who is intended to receive the transferred embryo, the level of soluble MICA is determined prior initiation of hormonal conditioning treatment and a level of MICA greater than 2.45 ng/ml in the serum sample is indicative of higher implantation failure rates. In a second particular embodiment, prior initiation of hormonal conditioning treatment, a level of MICA greater than 28 ng/ml in the serum sample is indicative of a high probability of IVF failure. Accordingly, the present invention concerns a method wherein the subject is the woman who is intended to receive the transferred embryo, the level of soluble MICA is determined prior initiation of hormonal conditioning treatment and a level of MICA greater than 28 ng/ml in the serum sample is indicative of a high probability of IVF failure. In a third particular embodiment, prior initiation of hormonal conditioning treatment, a level of MICA greater than 6 ng/ml in the serum sample is indicative of a high probability of miscarriage. Accordingly, the present invention concerns a method wherein the subject is the woman who is intended to receive the transferred embryo, the level of soluble MICA is determined prior initiation of hormonal conditioning treatment and a level of MICA greater than 6 ng/ml in the serum sample is indicative of a high probability of miscarriage.

The level of soluble MICA protein can also be determined after implantation. Therefore, in a fourth particular embodiment, after implantation, a level of MICA greater than 3.2 ng/ml in the serum sample is indicative of a high probability of miscarriage. Accordingly, the present invention concerns a method wherein the subject is the woman who receives the transferred embryo, the level of soluble MICA protein can also be determined after implantation and a level of MICA greater than 3.2 ng/ml in the serum sample is indicative of a high probability of miscarriage.

In a fifth particular embodiment, a low level of MICA is indicative of a high probability of a successful IVF. In particular, prior initiation of hormonal conditioning treatment, a level of MICA in the serum sample lower than 28 ng/ml, preferably lower than 6 ng/ml and more preferably lower than 2.45 ng/ml, is indicative of a successful IVF, preferably a high probability of successful IVF.

Therefore, the present invention concerns a method for selecting a subject suitable for a IVF comprising *in vitro* assaying for MICA in a subject sample and selecting the subject having a level of MICA indicative of a successful IVF probability. The method can comprise a previous step of providing a sample from the subject. The subject is in particular a woman which is candidate for IVF. The sample can be selected from the group consisting of a sample of blood, plasma, serum, endometrium biopsy, uterine fluid, vaginal and cervical secretions, cervical mucus, Douglas' pouch, and peritoneal fluid in case of ceolioscopic exploration (endometriosis, adherent pelvis, infectious sequela). In a preferred embodiment, the sample is a sample of blood, plasma, or serum, preferably a serum sample. The sample can be used directly or can be subjected to previous treatments.

In a preferred embodiment of the method for selecting a subject suitable for a IVF, the method comprises assaying for soluble MICA protein in the sample. The level of soluble MICA is preferably determined prior initiation of hormonal conditioning treatment. For instance, a soluble MICA level in the serum sample lower than 28 ng/ml, preferably lower than 6 ng/ml, and more preferably lower than 2.45 ng/ml, is indicative of a probability of a successful IVF. In another preferred embodiment, the method comprises assaying for cell-free nucleic acid encoding MICA. By "cell-free" is intended the nucleic acid which is not contained into a cell. In an additional preferred embodiment, the method comprises an indirect MICA assaying and comprises assaying for anti-MICA antibodies present in the sample. The level of MICA in the sample is preferably determined prior initiation of hormonal conditioning treatment.

In an aspect of the disclosure, the subject is the man offering the semen. In this aspect, the sample can be selected from the group consisting of a sample of blood, plasma, serum and semen. The semen can be selected from the group consisting of fresh ejaculated semen, fresh semen after preparation for spermatozoid capacitation, epididymal semen before or after freezing, and testicular semen before or after freezing. Indeed, the presence of MICA in the sample is indicative of a lower fertilizing capacity. In a preferred aspect, the method comprises assaying for soluble MICA protein in the sample. In another preferred aspect, the method comprises assaying for cell-free nucleic acid encoding MICA.

Therefore, the present disclosure also relates to a method for determining semen or spermatozoid quality by measuring the level of MICA in a blood, plasma, serum or semen sample of a man offering the semen. The method can comprise a previous step of providing a sample from the subject. In a preferred aspect, the method comprises assaying for soluble MICA protein in the sample. In another preferred embodiment, the method comprises assaying for cell-free nucleic acid encoding MICA. In particular, the semen or spermatozoid quality is used to determine its fertilizing capacity. Preferably, the level of MICA in the sample is indicative of the quality of the semen or spermatozoid, and thereby the probability of successful IVF. Preferably the higher is the level of MICA, the lower is the quality of the semen or spermatozoid, and thereby the probability of successful IVF.

The present disclosure further relates to a method for selecting the semen or spermatozoid suitable for IVF of an oocyte, comprising measuring the level of MICA in a blood, plasma, serum or semen sample of a man offering the semen. The method can comprise a previous step of providing a sample from the subject. Indeed, the presence of MICA has been associated with an implantation failure. Preferably, the sample showing the lowest level of MICA is selected for IVF. In a preferred aspect, the method comprises assaying for soluble MICA protein in the sample. In another preferred aspect, the method comprises assaying for cell-free nucleic acid encoding MICA.

In addition, the present disclosure relates to a method for determining the probability of male infertility in a subject, comprising measuring the level of MICA in a blood, plasma, serum or semen sample from the subject. The method can comprise a previous step of providing a blood, plasma, serum or semen sample from the subject. Indeed, the presence of MICA has been associated with a male infertility. In a preferred aspect, the method comprises assaying for soluble MICA protein in the sample. In another preferred aspect, the method comprises assaying for cell-free nucleic acid encoding MICA.

In another aspect, the subject is the woman who provides the oocyte. The subject can also be the receiving woman. Indeed, the presence of MICA can be useful to assess the quality of the oocyte, and thereby of the embryo, the presence of MICA being indicative of a lower probability of viability and/or of implantation. In a preferred aspect, the sample is selected from the group consisting of a sample of blood, plasma, serum, endometrium biopsy, uterine fluid, vaginal and cervical secretions, cervical mucus, Douglas' pouch, follicular fluid, and peritoneal fluid in case of ceolioscopic exploration (endometriosis, adherent pelvis, infectious sequela). In particular, the sample can be a follicular fluid, for instance a follicular fluid obtained by a follicule puncture after oocytes harvest. This fluid can be provided by vaginal puncture after ovulation induction by HCG.

The sample can also be provided from embryonic supernatant or embryo culture medium. In a particular embodiment, the supernatant or the culture medium comes from embryos aiming to be transferred or to be freezed.

The sample can be used directly or can be subjected to previous treatments such as freezing, purification, heating, concentration, dilution, etc...

Therefore, the present disclosure also relates to a method for determining embryo quality by measuring the level of MICA in the embryo culture medium or supernatant. The level of MICA is measured at least 44-46 hours post fertilization, preferably 60, 70, 80 or 90 hours post fertilization. In a preferred aspect, the method comprises assaying for soluble MICA protein in the sample. In another preferred aspect, the method comprises assaying for cell-free nucleic acid encoding MICA. In particular, the embryo quality is used to determine the potential for successful implantation of an embryo. Preferably, the level of MICA in the embryo culture medium or supernatant is indicative of the quality of the embryo, and thereby the probability of successful implantation of the embryo. Preferably the higher is the level of MICA, the lower is the quality of the embryo, and thereby the probability of successful implantation of the embryo.

The present disclosure also relates a method for selecting an embryo suitable for embryo transfer, in vitro fertilization, or implantation, the method comprising measuring the level of MICA in the embryo culture medium or supernatant. In particular, the level of MICA is indicative of the suitability of the embryo for embryo transfer, in vitro fertilization, or implantation. Preferably, the embryo showing the lowest level of MICA in the culture medium is selected for embryo transfer, in vitro fertilization, or implantation. Indeed, the presence of MICA has been associated with an implantation failure. In a preferred aspect, the method comprises assaying for soluble MICA protein in the sample. In another preferred aspect, the method comprises assaying for cell-free nucleic acid encoding MICA.

The present disclosure further relates to a method of IVF comprising selecting an embryo with a method of the present disclosure and transferring the embryo into a compatible human uterus.

In an other aspect, the present invention concerns methods for determining the probability of pregnancy complications, including miscarriage, vascular pregnancy diseases, preeclampsia, vascular or non-vascular intra-uterine growth retardation (IUGR), associated or not with preeclampsia, HELLP syndrome, gravidic steatosis, gravidic nephropathy or intra-uterine foetal death, in a subject comprising *in vitro* assaying for MICA in a sample, the level of MICA being indicative of a probability of pregnancy complications, including miscarriage, vascular pregnancy diseases, preeclampsia, vascular or non-vascular intra-uterine growth retardation (IUGR), associated or not with preeclampsia, HELLP syndrome, gravidic steatosis, gravidic nephropathy or intra-uterine foetal death. In particular, the present method is useful for determining the severity of preeclampsia. Accordingly, the present invention concerns a method for determining the probability of severe preeclampsia in a subject comprising *in vitro* assaying for MICA in a sample, the level of MICA being indicative of a probability of severe preeclampsia.

The method can comprise a previous step of providing a sample.

The sample used in this method can be selected from the group consisting of the blood, plasma, serum, placenta, cord's blood, endothelial cells and amniotic fluid samples. In a preferred embodiment, the sample is a sample of blood, plasma, or serum, preferably a serum sample. The sample can be used directly or can be subjected to previous treatments.

The subject is preferably a pregnant woman or a woman wishing to be pregnant.

In a preferred embodiment, the method comprises assaying for soluble MICA protein in the sample. In another aspect of the disclosure, the method comprises assaying for cell-free nucleic acid encoding MICA. In an additional aspect of the disclosure, the method comprises an indirect MICA assaying and comprises assaying for anti-MICA antibodies present in the sample.

Preferably, the level of soluble MICA is determined after embryo implantation. Preferably, the detection of soluble MICA in a serum sample, for instance at least 0.3 ng/ml, 0.5 ng/ml, 0.75 ng/ml or 1 ng/ml, is indicative a higher probability of pathologic pregnancy, including miscarriage, vascular pregnancy diseases, preeclampsia, vascular or non-vascular intra-uterine growth retardation (IUGR), associated or not with preeclampsia, HELLP syndrome, gravidic steatosis, gravidic nephropathy or intra-uterine foetal death, more particularly a pathology selected from the group consisting of VPD, preeclampsia, vascular IUGR, IUFD and a combination thereof. Preferably, the detection of soluble MICA in a serum sample, for instance at least 0.3 ng/ml, 0.5 ng/ml, 0.75 ng/ml or 1 ng/ml, is also indicative a higher probability of severe preeclampsia, in particular preeclampsia associated with proteinuria reflecting renal failure and/or bilateral early diastolic uterine notch which is a reflection of placental insufficiency. In a particular embodiment, a level of soluble MICA protein in a serum sample greater than 2.5 or 10 ng/ml is indicative of a high probability of pathologic pregnancy, including miscarriage, vascular pregnancy diseases, preeclampsia, intra-uterine growth retardation (IUGR), associated or not with preeclampsia, HELLP syndrome, gravidic steatosis, gravidic nephropathy or intra-uterine foetal death.

The present invention also concerns a method for differentiating vascular from non vascular intra-uterine growth retardation (IUGR) in a subject comprising *in vitro* assaying for MICA in a sample, the level of MICA being indicative of a probability of vascular IUGR. Preferably, the sample is a sample of blood, plasma, or serum, preferably a serum sample. More preferably, the detection of soluble MICA in the sample, preferably at least 0.3 ng/ml, 0.5 ng/ml, 0.75 ng/ml or 1 ng/ml, is indicative a vascular IUGR. Preferably, the level of soluble MICA is determined after embryo implantation.

As indicated above, the step of assaying MICA can comprise assaying the soluble MICA protein in the sample. In a preferred embodiment, the step of assaying for soluble MICA protein in the fluid sample comprises contacting the sample with an anti-MICA antibody. In a preferred embodiment, the step of assaying for soluble MICA in the sample is performed by ELISA assay, preferably by sandwich ELISA assay. The ELISA techniques for analysis and quantification of proteins, especially soluble proteins, are well known by the man skilled in the art. In an alternative embodiment, the step of assaying for soluble MICA in the sample is performed by coupling of antibody to microbeads (Luminex technology), Western blot, dot blot, radioimmunoassay (RIA), FACS analysis and other immunoassay techniques well known by the man skilled in the art.

For instance, a ELISA assay can be performed as following: a test sample is immobilized on wells of a polystyrene microtitre plate; anti-MICA antibodies are added to the wells; after binding and washing to remove non-specifically binding, the bound anti-MICA antibodies are detected. Detection is often achieved by the addition of a detectable antibody specific of anti-MICA antibodies. For instance, it can be detected by a third antibody directed against the Fc part of the second antibody (for instance, an anti-mouse IgG antibody from goat). Alternatively, the anti-MICA antibody can be labelled.

Preferably, a sandwich ELISA assay can be performed as following: anti-MICA antibodies are immobilized on wells of a polystyrene microtitre plate; a test sample is added to the wells; after binding and washing to remove non-specifically binding, the bound MICA is detected by a second anti-MICA antibody. Detection is often achieved by the addition of a detectable antibody specific of anti-MICA antibodies. For instance, it can be detected by a third antibody directed against the Fc part of the second antibody (for instance, an anti-mouse IgG antibody from goat). Alternatively, the second anti-MICA antibody can be labelled.

The anti-MICA antibodies useful in the present invention can be polyclonal or monoclonal. Antibodies can be of any class, preferably IgG1 or IgG2a. They can be specific of MICA, in particular soluble MICA. These antibodies are able to bind the extra-cellular part of MICA. Alternatively, they can bind both MICA and MICB.

Means for preparing and characterizing antibodies are well-known in the art (Harlow and Lane, Antibodies: A Laboratory Manual., Cold Spring Harbor Laboratory Press, Cold Spring Harbor, NY (1988)). More specific examples of antibodies are the following : 2C10, 6D4, 6G6 and 3H5 in WO 03/089616, and SR99, SR104 and SR116 in Hue et al (2003). Preferably, the anti-MICA antibody is a monoclonal antibody, preferably selected from the group consisting of SR99, SR104 and SR116.

The anti-MICA antibodies can be labelled. The label can be radioactive, fluorescent, chemilluminescent, an enzyme, or a ligand. The anti-MICA antibodies can also be unlabelled and may be used in conjunction with a detection agent that is labelled. For instance, they can be detected by a second antibody directed against the Fc part of the anti-MICA antibody (for instance, an anti-mouse IgG antibody from goat).

In other aspect of the disclosure, the step of assaying MICA can comprise assaying for cell-free nucleic acid encoding MICA. The nucleic acid encoding MICA can be RNA or DNA. The Genbank entry for the mRNA encoding Homo sapiens MICA is NM_000247. The GeneID for Homo sapiens MICA is 4276. Such cell-free nucleic acid encoding MICA can be free in the fluid sample and/or comprised into circulating microparticles, in particular microparticles present in blood or serum samples. Nucleic acid encoding MICA can be assayed by any means well-known by the man skilled in the art. For instance, it can be assayed by real time quantitative PCR, RT-PCR, by Southern or Northern blot or a combination thereof by using suitable primers and probe. In a preferred aspect, it is assayed by quantitative PCR or RT-PCR. The one skilled in the art can easily design suitable primers pair. For example, a primers pair suitable for assaying MIC A RNA by quantitative RT-PCR by sybergreen can be the following : MICA 970F and MICA 1127R.

| Primer | Position from ATG | Sens | Position | Sequence | Tm Primer express | Tm Primer 3 | Etot | E3' | Product length | Product Tm | E anyl tot | E anyl 3' | PCR Conditions |
|---|---|---|---|---|---|---|---|---|---|---|---|---|---|
| MICA 970F | 931 | F | Exon 5 | SEQ ID No 1 | 60,6 | 62,8 | 4 | 2 | 158 | 87,4 | 3 | 1 | 62° 7" |
| MICA 1127R | 1088 | R | Exon 7 | SEQ ID No 2 | 61,3 | 63,7 | 4 | 1 | | | | | |

The nucleic acids of the sample can be purified or enriched before to apply these techniques. The detection of nucleic acids encoding MICA has been extensively described in patent application WO 98/19167, the disclosure of which is hereby incorporated by reference.

In other aspect of the disclosure, the step of assaying MICA can comprise for anti-MICA antibodies present in the sample. For instance, the anti-MICA antibodies can be detected by ELISA assay using MICA proteins, preferably recombinant MICA protein. Methods for preparing such MICA proteins have been extensively described in patent applications WO 98/19167 and WO 03/089616, the disclosure of which is hereby incorporated by reference. Alternatively, the step of assaying for anti-MICA antibodies in the sample can be performed by coupling of MIC, preferably MIC A, to microbeads (Luminex technology). A commercially available kit Labscreen (One Lambda, ref LSMICA001) is useful for assaying anti MIC antibodies in a serum sample by Luminex. Such an assay for anti-MIC antibodies can be effective for the autoantibodies assay in autoimmune pathology associated to infertility (coeliac disease, APS, ...). This assay can also be valuable in case of women having a gravidic pathology with immunologic etiology (preeclampsia, habitual abortion).

In addition, the present invention disclosure relates to a method for determining the probability of infertility in a subject suffering of an autoimmune disease or disorder, comprising measuring the level of MICA in a sample from the subject, the level of MICA being indicative of a probability of infertility for the subject. Optionally, the method can comprise a previous step of providing a sample from the subject. The sample can be selected from the group consisting of blood, plasma, serum, endometrium biopsy, uterine fluid, vaginal and cervical secretions, cervical mucus, Douglas' pouch, follicular fluid, peritoneal fluid in case of ceolioscopic exploration, and semen. In a preferred aspect, the method comprises assaying for soluble MICA protein in the sample. In another preferred aspect, the method comprises assaying for cell-free nucleic acid encoding MICA. In an additional preferred embodiment, the method comprises assaying for anti-MICA antibodies present in the sample.

The present disclosure also relates to the use of a kit comprising at least one element selected from the group consisting of an anti-MICA antibody, a set of primers specific of a nucleic acid encoding MICA, a probe specific of a nucleic acid encoding MICA and a MICA protein in the medicine and biology of the reproduction. It also concerns a kit for the medicine and biology of the reproduction comprising at least one element selected from the group consisting of an anti-MICA antibody, a set of primers specific of a nucleic acid encoding MICA, a probe specific of a nucleic acid encoding MICA and a MICA protein. The MICA protein can be a soluble MICA. The kit can further comprise a leaflet, any suitable negative and positive controls, standard protein and/or detection reagent.

In a preferred embodiment, the kit comprises an anti-MICA antibody. The kit can further comprise a soluble MIC protein, preferably a MICA protein. Soluble MICA can be produced by recombinant expression of a truncated MICA. Soluble MICA can be expressed from suitable host cells, such as bacteria, yeast, mammalian and insect cells. To facilitate purification, a tag such as Myc or His tag can be included in the coding sequence. Such soluble MICA can be useful for the positive controls. Recombinant soluble MICA molecules can be prepared as described in Hue et al, 2003 (p. 1910), the disclosure of which is incorporated herein by reference. The kit can further comprise a microtitre plate, optionally having an anti-MICA antibody immobilized on the microtitre plate wells. Finally, the kit can comprise any suitable immunodetection reagents or solvant. The present invention also concerns in particular the use of the kit for the probability of *in vitro* fertilization (IVF) failure, embryo implantation failure, miscarriage, intra-uterine growth retardation and preeclampsia. Preferably, the anti-MICA antibody is a monoclonal antibody, preferably selected from the group consisting of SR99, SR104 and SR116. In a particular embodiment, the kit further comprises a soluble MICA protein.

The present invention also concerns the use of a kit according to the present invention for selecting a subject suitable for a IVF, for determining *in vitro* fertilization (IVF) outcome, for determining semen or spermatozoid quality, for selecting the semen or spermatozoid suitable for IVF, for determining the probability of male infertility in a subject, for determining embryo quality, for selecting an embryo suitable for embryo transfer, in vitro fertilization, or implantation, for identifying a subject having a high complication risk during pregnancy, in particular a subject susceptible to miscarriage, vascular pregnancy diseases, preeclampsia, intra-uterine growth retardation (IUGR), associated or not with preeclampsia, HELLP syndrome, gravidic steatosis, gravidic nephropathy or intra-uterine foetal death, for determining the severity of preeclampsia, for differentiating vascular from non vascular intra-uterine growth retardation (IUGR) and for determining the probability of infertility in a subject having an auto-immune disease, in particular a celiac disease or an antiphospholipid antibody syndrome (APS).

The present invention also concerns a kit for assaying soluble MICA comprising at least one anti-MICA antibody selected from the group consisting of SR99, SR104 and SR116. Preferably, the kit comprises two anti-MICA antibodies, in particular SR99 and SR104. Optionally, one of the two antibodies is labelled (e.g. biotinylated antibody). In a preferred embodiment, the kit is suitable for sandwich ELISA assay. The present invention further concerns the use of this kit in a method according to the present invention.

In addition, the assaying of MICA in any one of the methods and uses of the present invention can be combined with the assaying of other markers known in the art. The combination of several markers can still increase the predictability. For instance, these additional markers can be selected in the group consisting of HLA-G and angiogenic markers such as endoglin, PIGF and sFLT1. In order to assay these additional markers, the kit can comprise one or several additional elements selected from the group consisting of an antibody, primers, and probes specific of one or more additional markers such as a HLA-G, endoglin, PIGF and/or sFLT1 antibody, a set of primers specific of a nucleic acid encoding HLA-G, endoglin, PIGF or sFLT1, and/or a probe specific of a nucleic acid encoding HLA-G, endoglin, PIGF or sFLT1.

Further aspects and advantages of the present invention will be disclosed in the following experimental section, which should be regarded as illustrative.

### Examples

### Example 1 : serum levels of soluble MIC are predictive markers of implantation failure and successful term pregnancies following in vitro fertilization.

Uterine NK (uNK) are the predominant lymphoid cell population found at the embryo implantation site and progressively disappear after mid-gestation. Innate immune mechanisms operating in the mother are thus thought to have a strong influence in acceptance of the semi-allogeneic foetus. In particular, uNK receptors recognize paternal/trophoblast ligands, which prevent foetal attack by the maternal immune system.

Presence of soluble HLA-G, a known NK inhibitory ligand usually expressed at the fetomaternal interface, in embryo supernatants, has been correlated to higher embryo implantation rates after *in vitro* fertilization (IVF) (Fuzzi *et al.,* 2002; Warner *et al.,* 2004). Interaction between maternal killer immunoglobulin receptors (KIR) on uNK and foetal HLA-C also influences reproductive success (Hiby *et al.,* 2004; Parham, 2004; Wu *et al.,* 2004). In addition, uNK cells secrete angiogenic factors and cytokines that favour implantation and placentation (Ashkar *et al.,* 2003; Coulam *et al.,* 2003; Hanna *et al.,* 2006; Ledee-Bataille *et al.,* 2004; Moffett-King, 2002).

Therefore, the inventors deduced that any dysfunction of uNK cells should thus represent a drawback to successful implantation and pregnancy.

Functional implications of the expression of sMIC stress-inducible MHC class I related molecules has been widely explored in oncology (Carbone *et al.,* 2005; Groh *et al.,* 2005; Groh *et al.,* 1998) and auto-immunity (Hue *et al.,* 2004; Meresse *et al.,* 2004). Stress induced NKG2D ligands expression on allogeneic or autologous cells has been shown to target NK cell cytotoxicity and cytokine/chemokine production (Andre *et al.,* 2004; Lanier, 2005; Meresse *et al.,* 2004; Raulet *et al.,* 2003; Sutherland *et al.,* 2002). Dual activatory and stimulatory mechanisms have been associated to engagement of soluble MIC by the NKG2D receptor. Indeed, the release of a soluble form of MIC (sMIC) in the serum of some cancer patients has been shown to induce intemalisation of the stimulatory NKG2D receptor in effector NK and T lymphocytes, thus impairing both innate and adaptive anti-tumour immune responses and an escape mechanism favouring tumour growth (Groh *et al.,* 2002; Wu *et al.,* 2004). Such down regulation of NKG2D by placental-derived SMIC has also recently been suggested as an immune escape mechanism that may down regulate maternal immune responses during pregnancy (Mincheva-Nilsson *et al.,* 2006).

However, sMIC stress-inducible MHC class I related molecules has never been investigated with regards to reproduction failure. For the first time, the inventors investigated whether serum sMICA could be evidenced in women that fail to become pregnant or to give birth to a viable baby after in vitro fertilization (IVF).

### Materials and Methods

### Subjects and Methods

The present prospective study, approved by the local ethics committee, was performed on a consecutive series of infertile patients who underwent an IVF or ICSI at the Center of Assisted Reproductive Medicine, La Conception Hospital in Marseilles (January 2004- October 2005). A cohort of 170 infertile women, all candidates for IVF, was recruited for this study after given consent. Clinical indications were unexplained infertility, male infertility and tubal factor. None of the women included had a history of previous pregnancy. Plasma samples were collected during the follicular phase of the cycle preceding IVF. All patients received a similar stimulation regimen. Ovarian stimulation was performed by using recombinant FSH (Gonal F®, Serono Pharma, Paris, France ; Puregon®, Organon France, Paris, France) started after pituitary down regulation with Gnrh agonist analog. Complete pituitary desensitization was confirmed by both low plasma oestradiol below 50 pg/ml and ultrasound examination to exclude ovarian cyst and confirmed the endometrial thickness below than 5mm. Human chorionic gonadotrophin 10000 UI (HCG) was administered when at least three follicles exceeded 16mm in diameter. Oocyte recovery was performed by transvaginal ultrasound guidance and general anesthesia 32-34 hours after HCG administration. Luteal phase was supported with natural progesterone from the day of embryo transfer. The embryo transfer was performed on the 2nd or 3rd day post-oocyte collection. A single serum HCG measurement was performed 15 days after embryo transfer. A clinical pregnancy was defined when an intra uterine gestational sac with fetal heartbeat was detected by transvaginal ultrasonography.

Collected oocytes were cultured in a four-well multi-dish with 600 µL of culture medium added with serum substitute supplement. Each well contained from one to four oocytes. IVF or ICSI technique was used for insemination. Oocytes fertilization was observed 16-18h after insemination under an inverted microscope and fertilization rate was calculated. Embryos were examined after 48h or 72h in culture, the rate of cleavage was assessed and up to four embryos were chosen for transfer. The embryos were graded according to the number of blastomers and the amount of fragmentation. The grades used were: grade 1 (no fragments), grade 2 (<20%), grade 3 (20-50% fragmentation), grade 4 (>50%fragmentation).

### ELISA assay of soluble MIC levels in plasma

Construction, expression, and purification of soluble MICA molecules : Recombinant soluble MICA molecules have been prepared as described in Hue et al, 2003 (p. 1910), the disclosure of which is incorporated herein by reference.

Production of anti-MICA mAbs : Anti-MICA monoclonal antibodies have been produced as described in Hue et al, 2003 (p. 1910), the disclosure of which is incorporated herein by reference.

Detection of soluble MICA by ELISA : To detect soluble MICA in the serum, two different anti-MIC mAbs were used in a sandwich ELISA. High-binding polystyrene plates (Greiner; Sigma-Aldrich) were coated with the capture SR99 Ab (5 µg/ml in PBS, 100 µl per well) for 12 h at 4°C, washed five times with PBS plus 0.05% Tween 20, blocked by addition of 100 µl of 5% BSA for 1 h at 22°C, and washed in PBS-0.05% Tween 20. The standard (serial dilutions of soluble rMICA from 100 µg/ml to 0.1 ng/ml in PBS-0.05% Tween 20) and the serum samples (100 µl per well) were then added for 2 h at room temperature. After five washes, the biotinylated SR104 detection Ab (150 ng/ml in PBS, 100 µl per well) was added for 1 h at room temperature. Plates were washed and incubated for 20 min at room temperature with streptavidin- conjugated HRP (1/30,000; Amersham Pharmacia Biotech), washed, and reacted with tetramethylbenzidine substrate (Sigma-Aldrich) for 15 min at 37°C. Reaction was stopped with 0.5 M H₂SO₄ (50 µl per well). The absorbance was measured at 450 nm. The detection threshold of recombinant soluble MICA protein was 0.1 ng/ml.

### Statistical analysis

Differences between groups were evaluated for statistical significance by one-way ANOVA, student's t test or Mann-Whitney rank sum test, depending on whether the data were normally distributed, using the GraphPad Prism software version 4.0b. Chi-Square analysis was used to compare sMIC frequencies between various independent groups. Receiver-operator characteristic curve analysis (ROC) was used to analyse SMICA concentration cut-off points and their sensitivity/specificity. Cut-off determined further used to evaluate relative risks and odds ratio. Significative p values were set as <0.05.

### Results

### Higher sMIC serum levels are found in infertile women that experience implantation failure after IVF

Of the 170 patients included, 38 experienced successful embryo implantation following IVF, among which 30 ongoing pregnancies gave birth to a viable baby at term and 8 miscarriages occurred after successful implantation. Three patients experienced miscarriage at 8 SA, two patients at 9 SA, two patients at 6 SA and one patient at 7 SA. Pathology reports (realised for > 8 SA losses) were not in favour of genetic losses. Patient's characteristics are summarized in Table 1.

Serum sMIC was measured with using a sandwich ELISA as described (Hue *et al.,* 2004). Sixty-four (38 %) patients undergoing IVF had detectable sMIC serum levels (mean = 15.28 ng/ml, lower-upper 95 % ranges = 10.7-19.9 ng/ml).

Despite the fact that parameters associated to IVF outcome where normalized in these groups (Table 1), mean sMIC levels in positive patients significantly differed in women that had successful embryo implantation (median=2.5, 25-75% percentile ranges: 1.5-9.7) as compared to the implantation failure group (12.0, 3-24.2, p<0.021). The inventors thus further investigated whether sMIC levels values may help prediction of implantation failure in sMIC positive women. They thus defined a 2.45 ng/ml cut-off value allowing risk evaluation by ROC analysis (sensitivity 82, specificity 50%). sMIC> to 2.45 were associated to implantation failure (odds ratio: 4.6, 95% CI 1.08-19.79, p=0.031). sMIC serum levels > 27.7 ng/ml were always associated with lack of embryo implantation after IVF (Fig. 1).

**Table 1 : Characteristics of the 170 infertile women recruited in the study**

| | **Non Pregnant Implantation Failure** | **Miscarriage After Implantation** | **Evolutive pregnancy Live Born Baby** | ***P- value*** |
|---|---|---|---|---|
| **Number of patients** | 132 | 8 | 30 | *NA* |
| **Age (years) (mean ± sd)** | 32.07±3.742 | 32.00±3.721 | 32.51±3.373 | *0.88* |
| **Tubal factors (%)** | 29.25 | - | 24.6 | *ns* |
| **Male factors (%)** | 41.25 | 66 | 44 | *ns* |
| **Unexplained (%)** | 29.5 | 34 | 21.4 | *ns* |
| **Duration of infertility (years) (mean ± sd)** | 4.38±2.221 | 4.37±2.12 | 4.64±3.58 | *0.67* |
| **Basal FSH levels (UI/I) (mean± sd)** | 7.15±2.19 | 7.23±1.86 | 6.99±2.85 | *0.93* |
| **Basal LH levels (UI/I) (mean ± sd)** | 4.94±1.37 | 4.77±1.10 | 4.45±1.46 | *0.84* |
| **Gonadotrophin (UI)** | 2120±962.9 | 2125±831.6 | 2299±678.2 | *0.63* |
| **Endometrium (mm)** | 10.60±1.91 | 11.40±0.89 | 10.86±1.86 | *0.69* |
| **Duration of stimulation (days)** | 12.12±1.68 | 10.42±1.92 | 11.74±2.37 | *0.52* |
| **Estradiol (UI) on HCG day** | 2001±939.5 | 2268±1391 | 1912±1018.4 | *0.65* |
| **Number of oocytes collected (mean ± sd)** | 9.5±5.41 | 8.25±3.19 | 10.2±4.49 | *0.61* |
| **Fertilization rate (%)** | 77.25±16.92 | 72.56±31.62 | 74.13±16.76 | *ns* |
| **Number of embryos available for transfer (mean** ± **sd)** | 6.77±3.74 | 6.12±3.98 | 7.13±3.53 | *0*.*75* |
| **Mean of embryos transferred** | 2.044±0.41 | 1.8±0.42 | 2.0±0 | *0.25* |

### sMIC serum levels predicts chances to have a viable born baby at term after IVF

In addition to predict implantation failure, the inventors further prospectively assayed whether increased sMIC levels could predict IVF outcome as birth of a live baby. Indeed after implantation was successful (38 women), 8 women experienced a miscarriage after IVF and 30 gave birth to a viable baby. When positive, soluble serum MIC values observed before IVF were found to be significantly lower in the group of women that gave birth after IVF (median= 2 ng/ml, min.- max. ranges: 1-6ng/ml) in reference to women who experienced miscarriage or implantation failure after IVF (median=11 ng/ml, min-max: 0.1-96 ng/ml, Fig. 1). The inventors thus further evaluated the value of sMIC that may predict chances of achieving successful pregnancy instead of miscarriage or implantation failure. Using the same cut-off of 2.45 ng/ml (sensitivity=82% and specificity = 75%), the chances to have a viable baby were higher when predicted by sMIC serum levels prior IVF that are lower than the 2.45 ng/ml threshold (odds ratio=13.8, CI 95%=2.03-118, p= 0.002). Highest values of sMIC observed in women achieving ongoing pregnancies were 6ng/ml (Fig.1).

### High Values of sMIC are associated to miscarriage after implantation success

Although the frequency of sMIC positive women did not significantly differ in relation with implantation success (36% in the implantation success versus 38% in implantation failure group), when implantation was successful (38 cases), frequencies of sMIC positive were higher in the group of women experimenting miscarriage (75%) than in that with successful term pregnancies (26%, p= 0.03). Further more, when positive, median sMIC serum levels prior IVF were shown to be higher in the group that experienced miscarriage after IVF (median=9.7, min.-max. observed = 2.5-24 ng/ml) than in the group successfully achieving pregnancy (median=2.1, min.-max ranges 1-6ng/ml. The inventors thus further evaluated whether the risk of miscarriage could be predicted from sMIC serum levels. While higher sMIC serum levels were not associated to occurrence of miscarriage in the total population of pregnant women in absence of IVF, the inventors could show that, when implantation is successful after IVF, higher sMIC were associated with miscarriage occurring during pregnancy obtained after IVF. Indeed, using a cut-off value of sMIC serum levels > 3.2 ng/ml (sensitivity 83% and specificity 75%), sMIC could predict miscarriage after successful implantation when compared to term pregnancy (Odds ratio= 35, p= 0.026, 95% CI 1.74- 703). No birth at term was observed when women had sMIC serum levels > 6ng/ml.

### Discussion

The mechanisms by which sMIC influences embryo implantation remains unclear. Natural killer cells play an essential role at early stages of implantation, as they represent the predominant immune partners controlling cytokine/chemokine/angiogenic factor production and trophobast modelling (Sargent *et al.,* 2006). sMIC is one of the numerous ligands for the activating NKG2D receptor, widely expressed on NK and CD8 and γδ T lymphocytes. The engagement of activating NKG2D receptors by NKG2D ligands, among which soluble MIC, is a co-stimulatory signal for NK-mediated cytotoxic activity, proliferation and cytokine production (Andre *et al.,* 2004; Bryceson *et al.,* 2006; Raulet *et al.,* 2003; Sutherland et al. , 2002; Upshaw *et al.,* 2006). Alternatively, sMIC-induced internalisation of its NKG2D receptor has been described as a mechanism down regulating anti tumoral NK cell activity (Wu *et al.,* 2004). Down regulation of NKG2D by placental-derived soluble MIC has recently been suggested as an immune escape mechanism favouring foetal survival (Mincheva-Nilsson *et al., 2006*). Thus, modulation of the uNK-cell cytokine repertoire or lytic activity, resulting from interaction between the NKG2D receptor and its ligands, may affect the implantation environment and be detrimental to successful pregnancy after IVF.

The main finding of the inventors is thus that the stress inducible immunostimulatory MHC class I Chain- Related molecule, is prevalent before IVF in women that will experience implantation and pregnancy failure. Serum levels of sMIC greater than 2.45 ng/ml are predictive of higher implantation failure rates matrix, and that sMIC serum levels > 6ng/ml never resulted in term evoluting pregnancy while levels > 28 ng/ml were always associated with IVF failure. Furthermore, after implantation is successful, women that bear sMIC levels > 3.2 ng/ml are at high risks of experimenting miscarriage after IVF. The origin and infertility status -related mechanisms that may contribute to enhanced sMIC protein levels in the serum of women that will not achieve successful pregnancy after IVF failure remain unravelled. The release of sMIC from the membrane of MIC-expressing cells have been reported to involve cleavage by metalloproteinase (Waldhauer *et al.,* 2006). Altered metalloproteinase activity has been reported in IVF patients with recurrent implantation failure (Shibahara *et al.,* 2005). Further investigation of the mechanisms generating soluble MIC and the impact of sMIC on immune functions associated with implantation failure or pregnancy loss after IVF are now challenging issues to explore. The fact that sMIC is also a marker of auto and alloimmune processes, as contributed by co-authors of this study in celiac disease (Hue *et al.,* 2004), where higher prevalence of infertility is reported (Meloni *et al.,* 1999), suggests sMIC may be the signature of a higher auto or alloreactive potential of the mother to reject the foetal embryo.

Altogether, considering high economic and psychological implications of IVF, this study is the first to provide arguments that seric sMIC quantification may be considered as a novel parameter with applications in the non-invasive evaluation and prediction of IVF outcome. Besides its prognosis value, a major value of this biomarker is the possibility of its dosage prior initiation of women hormonal conditioning treatment. These features should thus improve counselling or management of IVF associated-risks and thus provide consequent benefits for infertile women health care.

### Example 2 : Soluble MIC is found at higher frequencies in plasma of women Vascular pregnancy diseases.

Pregnancy creates a unique situation in which extensive angiogenesis and establishment of maternal immune tolerance allow successful fetal and placental development. Cross talk between trophoblast fetal cells and maternal immune cells regulate early pregnancy placental development, vasculogenesis and immune tolerance of the fetus. Inadequate placentation leads to vascular pregnancy diseases (VPD) that include preeclampsia (PE), intrauterine growth retardation (IUGR) and recurrent pregnancy loss, which represent a leading cause of fetomaternal morbidity and mortality. Preeclampsia is characterized by hypertension and proteinuria after 20 weeks of gestation. Despite recent advances in understanding how inadequate placental vascularization (incomplete spiral artery remodeling) may lead to vascular pregnancy diseases, their prognosis remains severe, and placental removal is still the only treatment to manage severe preeclampsia and major intra uterine growth restriction.

### Example 2a

### Materials and Methods

### Subjects

Soluble MIC plasma levels were evaluated in 49 women that experienced vascular pregnancy diseases (VPD) that include vascular Intrauterine growth retardation (IUGR), Preeclampsia (PE), or intra uterine foetal death (IUFD) and compared to a control group of plasma from 53 women with normal ongoing pregnancies matched for pregnancy term (mean 29 weeks).

### Methods

PE was defined as a diastolic arterial blood pressure greater than 90 mm Hg, and a systolic blood pressure greater than 140 mm Hg, associated with proteinuria (more than 300 mg/24h). In addition, severe maternal complications associated with increased risk of adverse outcome for both mother and fetus were reported, particularly association with HELLP syndrome (H = hemolysis; EL = elevated liver enzymes; and LP = low platelets) (Weinstein *et al.,* 2005) and oliguria that leads to renal failure.

Vascular IUGR was defined as ultrasonographic measurement <2.5^{th} percentile for gestational age associated with at least one biological or sonographical marker of "placental insufficiency" as abnormal uterine, or umbilical artery Doppler (Chien *et al.,* 2000), or elevated plasma fibronectin level (Ostlund *et al.,* 2001). Exclusion criteria of vascular IUGR group were the presence of congenital malformations or chromosomal abnormalities in the fetus, recent cytomegalovirus or toxoplasma infection, trauma, drugs or alcohol abuse during pregnancy and fetus constitutionally small for gestational age.

IUFD was defined by ultrasound examination as a visible fetus without cardiac activity after 12 weeks of gestation with fetal biometry according to term, occurring after severe growth retardation.

### Results

**Table 2 : Frequencies observed in the VPD group were higher than in a term matched control group.**

| | **Normal Pregnancies** | **Vascular pregnancy Diseases** | | | |
|---|---|---|---|---|---|
| | **NP** | **VPD** | **IUGR** | **PE** | **IUFD** |
| **n=** | 53 | 49 | 13 | 19 | 17 |
| **MIC+ n=** | 1 | 19 | 5 | 6 | 8 |
| **% MIC+** | 1,9 | 38,8 | 38,5 | 31,6 | 47,1 |
| **Mean** | 1.0 | 10.27 | 6.39 | 15.25 | 8.95 |
| **Std. Error** | 0.0 | 3.17 | 4.43 | 5.21 | 5.99 |
| *Minimum* | *1* | *0.35* | *0.4* | *1.37* | *0.35* |
| *Maximum* | *1* | *50.00* | *23.70* | *32.69* | *50.00* |

As shown in Figure 2, higher levels of MIC have been found in MIC+ positive samples of women with vascular pregnancy diseases.

### Example 2b

### Materials and Methods

### Subjects

Following informed consent, 169 women were consecutively included in the study conducted over a two-year period (2004-2006).
81 patients presented vascular pregnancy diseases (VPD) that were further subdivided in 3 groups: 40 patients with PE, 23 patients with vascular IUGR, 18 patients with intrauterine fetal death (IUFD). PE, IUGR, IUFD was defined as described above in the example 2a.
25 patients presented isolated non-vascular IUGR, related to chromosomal fetal aneuploidy in 4 cases, fetal polymalformative syndrome in 8 cases, toxic or infectious origin in 3 cases, and fetus small for gestational age in the 10 remaining cases .Non-vascular IUGR was diagnosed as ultrasonographic measurement < 2.5th percentile for gestational age with a normal fibronectin level, uterine and umbilical artery Doppler velocity.

The control group consisted of 63 healthy pregnant women seen for routine gynecologic examination and followed until delivery to confirm normal pregnancy (NP) outcome. Normal pregnancies were recruited between 17 and 41 weeks of gestation to match term of normal pregnancies with that of patients with VPD.

### Plasma collection

Blood samples were collected at time of diagnosis of vascular pregnancies diseases, or isolated IUGR and at time of obstetrical examination for the control group of term matched normal pregnancies. Samples were collected into 0.129 mol/L sodium citrate (3.8%) centrifuged and stored at -80° according to standard procedures.

### Capture ELISA for sMICAlB

Soluble MIC concentrations were measured in the plasma using a sandwich enzyme-linked immunoabsorbent assay as previously described (Hue *et al.,* 2004). The detection threshold of recombinant soluble MICA protein, used as standard in each experiment, was 0.1ng/ml and plasma levels higher than 0.3 ng/ml were considered as positive.

### Statistics

Analyses were performed with Prism software (GraphPad 4.0b, GraphPad, San Diego, CA) implementing the non parametric Kruskal-Wallis test followed by the Dunn post-test to compare 3 or more continuous variables, the Mann-Whitney test to compare 2 unpaired groups, and the Wilcoxon matched pairs test for the NKG2D expression and the CD107a degranulation assay results. Data were expressed as the median [range] and mean ( sd) depending on the distribution. Association between categorical variables was tested after cross tabulation by the Pearson chi square (and by fisher exact test if n<5). Correlation was improved by spearman test. A 95% confidence interval (p<0.05) was considered significant.

Multivariate analyses of parity, gestation, systolic and diastolic blood pressure, gestational age at sampling, term of delivery and baby weight at birth parameters were performed to identify independent marker associated to detection of soluble MIC in plasma.

### Results

### Increased frequency of plasma sMIC in women with vascular pregnancy diseases

Plasma levels of sMIC were evaluated in 3 groups of term-matched women with NP, VPD or non-vascular IUGR. No significant differences were found in age, number of gestation, body mass index and gestational age at sampling between normal pregnancies, VPD and non-vascular IUGR. The systolic and diastolic blood pressure was significantly higher in women with VPD. As expected the median of baby's birth weights and gestational age at delivery were significantly lower in the group with VPD and non-vascular IUGR compared to normal pregnancies (p<0.001). The main clinical and biological characteristics of these patients are summarized in Table 3.

**Table 3: Characteristics of the study population**

| | Normal pregnancies | Vascular pregnancy diseases | Non vascular IUGR | p value |
|---|---|---|---|---|
| Number of patients | 63 | 81 | 25 | |
| Age (yrs, mean±sd) | 29.6±6.6 | 30.2±6.3 | 29±6.3 | ns |
| Gestation (n) | 3[1-9] | 2[0-7] | 2[0-6] | ns |
| Parity (n) | 1[0-5] | 0[0-4] | 0[0-3] | ns |
| Body Mass Index (kg/cm²) | 23[17-48] | 23.1[16.3-37.2] | 23[18-37] | ns |
| Systolic Blood Pressure (mmHg) | 12[10-13] | 14[10-22] | 12[10-13] | p<0.001* |
| Diastolic Blood Pressure (mmHg) | 7[5-8.3] | 8.7[6-12] | 7[5.5-8.5] | p<0.001* |
| Uterine Height (cm) | 32[29-38] | 26[15-36] | 26[15-36] | p<0.001 § |
| Term at sampling (wks gest.) | 31.7[15.3-41] | 30[17-41] | 30[17-41] | ns |
| Term at delivery (wks gest.) | 40.3[35-42] | 32.3[17-41.1] | 32[22-41] | p<0.001 § |
| Baby weight at birth (g) | 3300[2640-4680] | 1330[80-3410] | 1745[400-2500] | p<0.001 § |

Comparison between groups was performed with non parametric Kruskal-Wallis test followed by the Dunn post-test. Values indicate median [25-75 interquartile ranges]
* p<0.001 between vascular pregnancy diseases and others groups
§ p<0.001 between Normal pregnancies and others groups

In the NP group, only one of the 63 women had low sMIC levels in plasma (0.5 ng/ml). However, it must be noted that this patient was followed for systemic lupus erythematosus, with normal pregnancy evolution. Of the 25 women with non-vascular IUGR, 1had detectable sMIC plasma levels (1.63 ng/ml). By contrast, sMIC molecules were detected in 26 of the 81 patients (32%) in the VPD group (p<0.0001 in reference to NP). Furthermore, sMIC levels were significantly higher in preeclampsia patients (median, 25-75 interquartile ranges: 7.5 ng/ml, 1.37-32.69) than in IUFD (2.18 ng/ml, 0.86-7.58, p<0.01) and vascular IUGR (1.63 ng/ml, 0.86-5.2, p<0.05) (Table 4).

**Table 4: Frequency of sMIC detection and plasma levels in the study population.**

| | Frequency of sMIC positive plasma Number (Proportion %) | Comparison of sMIC frequency in reference to control normal pregnancy group | Median sMIC plasma levels in positive samples (ng/ml) [25-75 interquartile range] |
|---|---|---|---|
| Normal pregnancies (n=63) | 1 (1.6%) | - | 0.5 |
| Vascular pregnancy diseases (n=81) | 26 (32%) | p<0.0001 | 2.2[1.15-11.47] |
| Preeclampsia (n=40) | 9 (22.5%) | p<0.0008 | 7.5[1.37-32.69] |
| Vascular IUGR (n=23) | 9 (39%) | p<0.0001 | 1.63[0.86-5.2] |
| IUFD (n=18) | 8 (44%) | p<0.0001 | 2.18[0.86-7.58] |
| Non vascular IUGR (n=25) | 1 (4%) | ns | 1.63 |

### The presence of plasma sMIC identifies a subgroup of severe preeclampsia.

It was then determined whether the presence of sMIC in maternal plasma was associated to clinical, biological or sonographical markers that characterize the severity of preeclampsia according to term at diagnosis, systolic or diastolic blood pressure, proteinuria quantification, uric acid and fibronectin plasma levels, umbilical and uterine artery Doppler velocity waveform analysis, delivery term and baby's birth weight (Table 3). There was no difference between sMIC-positive and -negative patients for systolic or diastolic blood pressure, fibronectin and uric acid level. However, the median proteinuria per day, which reflects renal failure, was significantly higher in PE women with sMIC-positive plasma than in PE women without detectable sMIC (p=0.04, Figure 3A). Multivariate analysis of parity, gestation, systolic and diastolic blood pressure, gestational age at sampling, term of delivery and baby weight at birth further confirmed that proteinuria was independently associated to presence of sMIC. In addition, sonographical analysis of maternal uterine artery Doppler velocity waveform performed at time of PE showed that bilateral early diastolic uterine notch, a reflection of placental insufficiency, was more frequent in the sMIC-positive group of PE (p=0.037, Figure 3B). The frequency of severe maternal complications among PE patients in relation with sMIC plasma detection was then studied. Among the 9 PE women with sMIC-positive plasma, 3 (33%) presented oliguria with acute renal insufficiency, compared to one of the 31 PE women (3.2%) without detectable plasma sMIC (p=0.03). Moreover, frequency of HELLP syndrome tended to be more elevated in the sMIC positive PE group (2 on 9 patients, 22.2%) when compared to the sMIC negative PE group (1 on 31 patients, 3.2%, p=0.1).

### The presence of sMIC in plasma is associated with vascular IUGR

To address whether the presence of plasma sMIC associates with the vascular origin of IUGR, the inventors compared sMIC levels in women with vascular and non-vascular IUGR. Among the 23 patients with vascular IUGR, 9 (39%) had detectable plasma sMIC, compared to only one out of the 25 (4%) with non-vascular IUGR (OR= 15.43 [1.763-135], p=0.0038). Thus while age, term at sampling, baby birth weight and term of delivery were not different between the 2 groups, the presence of sMIC in maternal plasma is significantly associated with vascular IUGR. Furthermore, positive sMIC plasma predicted the vascular etiology of IUGR with 90% positive predictive value (96% specificity and 39% sensitivity), and 63% negative predictive value.

### Discussion

The results of the inventors indicate that soluble MIC molecules are more frequently detected in plasma from women with vascular pregnancy diseases than in those with normal pregnancies matched for gestational age. Moreover, sMIC plasma levels are correlated with the severity of preeclampsia and appear to be a specific marker of vascular IUGR.

Preeclampsia is a multisystem disorder of unknown cause that is unique to human pregnancy. Among them, congenital thrombophilic defect have been associated with the occurrence of VPD. Novel inflammatory and thrombosis markers have been identified without clinical application (Levine *et al.,* 2006, Bretelle *et al.,* 2003 and 2005). Furthermore, high proteinuria is associated to increased risk of adverse maternal and fetal outcomes (Chan *et al.,* 2005). However proteinuria is not independently predictive of adverse outcome and no threshold value of protein excretion may predict severe preeclampsia complication. In the state of the art, no clinical data relevant marker of PE severity has been identified

Considering the results of the inventors, the presence of sMIC in maternal plasma provides a novel clinical relevant marker of PE severity. Optionally, this parameter could also be combined with other parameters in order to increase the level of sensitivity to predict preeclampsia, if needed.

The presence of sMIC in maternal plasma is also associated with IUGR, another subtype of vascular diseases of multiple origins, prognosis of which depends on the cause, severity and term of diagnosis. Placental insufficiency due to abnormal placentation constitutes the major cause of IUGR called vascular IUGR. Most vascular IUGR are associated with PE and share the same physiopathology. By contrast, isolated IUGR may represent the only sonographical sign of severe fetal infection, aneuploidy, or genetic syndrome associated with abnormal neurologic outcomes, so that patients may ask for pregnancy termination in such cases. Vascular IUGR is sometimes easily diagnosed in the presence of PE, maternal vascular markers (fibronectin, high uric acid level) or alteration of uterine Doppler Velocimetry waveflow. However, in the state of the art, there was a lack of non-invasive and specific markers that allow to differentiate vascular from non vascular IUGR. For example, indices of uterine Doppler velocimetry used in the prediction of placental hypoxic-ischemic lesions in IUGR show 63% specificity for 97% sensibility. In the absence of obvious IUGR etiology, invasive fetal explorations are required to determine fetal prognosis, with non-negligible rate of premature delivery and late fetal loss.

The results of the inventors show that sMIC detection in women plasma allows to predict the vascular origin of IUGR with 96% specificity. Therefore, the sMIC quantification at time of IUGR diagnosis constitutes a useful non-invasive tool to improve management of idiopathic IUGR.

These results show that quantification of sMIC molecules in women plasma is a useful non-invasive tool to predict the imminent onset of vascular complications during pregnancy.

### Example 3 : Soluble MIC is found at higher frequencies in follicular fluid, embryo supernatant, semen and in catheter used for embryo transfer in case of implantation failure.

Soluble MIC levels have been evaluated in follicular fluid (FF), transferred embryo supernatant (T embryo) or un transferred (UT) embryo supernatant, Semen used for IVF (T) or not (UT), and in catheter used for embryo transfer (KT). The presence of soluble MIC has been more frequently observed in case of implantation failure as shown in Table 5.

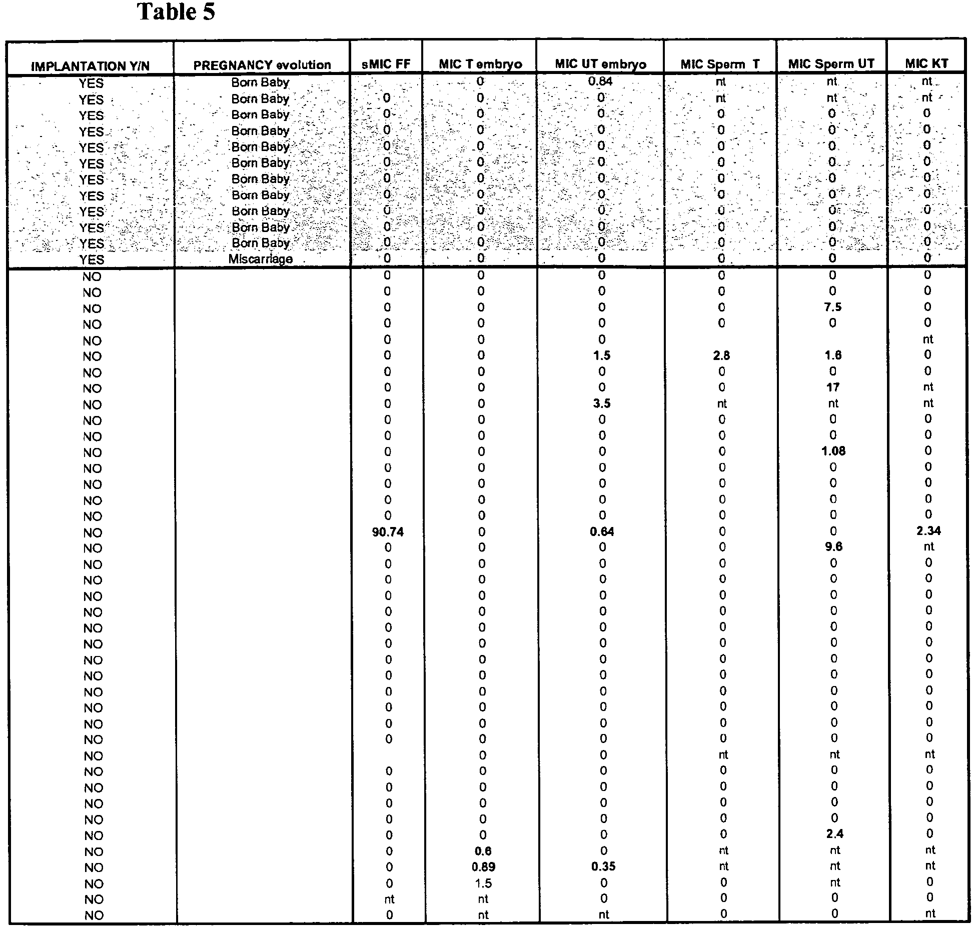

### References

Andre P, et al. (2004) Eur J Immunol 34, 961-971.
Ashkar AA, et al (2003) J Immunol 171, 2937-2944.
Bretelle, F., et al. (2003) Thromb Haemost 89:486-492.
Bretelle, F., et al. (2005) Am. J. of obstetrics and gynecology 193:1698-1702.
Bryceson YT, et al (2006) Blood 107, 159-166.
Carbone E, et al (2005) Blood 105, 251-258.
Chan, P., et al. (2005) Bjog 112:280-285.
Chien, PF, et al. (2000) Bjog 107:196-208.
Coulam CB and Roussev RG (2003) J Assist Reprod Genet 20, 58-62.
Fuzzi B, et al (2002) Eur J Immunol 32, 311-315.
Groh V, et al (2005) Proc Natl Acad Sci USA.
Groh V, et al (1998) Science 279, 1737-1740.
Groh V, et al (2002) Nature 419, 734-738.
Hanna J, et al. (2006) Nat Med. 12, 1065-74.
Hiby SE, et al (2004) J Exp Med 200, 957-965.
Hue S, et al. (2003) J. Immunol 171, 1909-1917.
Hue S, et al. (2004) Immunity 21, 367-377.
Lanier LL (2005) Annu Rev Immunol 23, 225-274.
Ledee-Bataille N, et al (2004) Fertil Steril 81, 59-65.
Levine, RJ., et al (2006) N Engl J Med 355:992-1005.
Meloni GF, et al (1999) Hum Reprod 14, 2759-2761.
Meresse B, et al. (2004) Immunity 21, 357-366.
Mincheva-Nilsson L, et al (2006) J Immunol 176, 3585-3592.
Moffett-King A (2002) Nat Rev Immunol 2, 656-663.
Ostlund, E, et al. (2001) Hypertens Pregnancy 20:79-87.
Parham P (2004) J Exp Med 200, 951-955.
Raulet DH (2003) Nat Rev Immunol 3, 781-790.
Sargent IL, et al (2006) Trends Immunol. 27, 399-404.
Shibahara H, et al (2005) Am J Reprod Immunol 54, 186-192.
Sutherland CL, et al (2002) J Immunol 168, 671-679.
Tong S, et al (2004) Lancet 363, 129-130.
Upshaw JL and Leibson PJ (2006) Semin Immunol 18, 167-175.
Waldhauer I and Steinle A (2006) Cancer Res 66, 2520-2526.
Warner CM, et al (2004) J Assist Reprod Genet 21, 315-316.
Weinstein, L. (2005) American J. of obstetrics and gynecology 193:860-863.
Wu JD, et al(2004) J Clin Invest 114, 560-568.

## Claims

1. A method for determining *in vitro* fertilization (IVF) outcome comprising *in vitro* assaying for soluble MICA (MHC class I chain-related protein A) in a blood, plasma, or serum sample from a woman who receives or is intended to receive a transferred embryo, the level of soluble MICA being indicative of the IVF outcome, in particular indicative of implantation failure rates, IVF failure, and/or miscarriage.

2. The method according to claim 1, wherein a low level of MICA is indicative of a high probability of a successful IVF.

3. The method according to claim 1 or 2, wherein the level of soluble MICA is determined prior initiation of hormonal conditioning treatment and a soluble MICA level in the serum sample lower 2.45 ng/ml, is indicative of a high probability of a successful IVF.

4. Method according to claim 1, wherein the sample is a serum sample and the subject is the woman who is intended to receive the transferred embryo, the level of soluble MICA is determined prior initiation of hormonal conditioning treatment, and
- a level of soluble MICA greater than 2.45 ng/ml in the serum sample is indicative of higher implantation failure rates; and/or
- a level of soluble MICA greater than 28 ng/ml in the serum sample is indicative of a high probability of IVF failure; and/or
- a level of soluble MICA greater than 6 ng/ml in the serum sample is indicative of a high probability of miscarriage.

5. Method according to claim 1, wherein the sample is a serum sample and the subject is the woman who receives the transferred embryo, the level of soluble MICA protein can also be determined after implantation and a level of MICA greater than 3.2 ng/ml in the serum sample is indicative of a high probability of miscarriage.

6. Method according to claim 1, wherein the method further comprises identifying the subject having a level of MICA indicative of a successful IVF probability.

7. Method according to claim 6, wherein the sample is a serum sample and the level of soluble MICA is determined prior initiation of hormonal conditioning treatment and a soluble MICA level in the serum sample lower than 28 ng/ml, preferably lower than 6 ng/ml, and more preferably lower than 2.45 ng/ml, is indicative of a high probability of a successful IVF.

8. A method for determining the probability of pregnancy complications, including miscarriage, vascular pregnancy diseases (VPD), preeclampsia (PE), severe preeclampsia, vascular intra-uterine growth retardation (IUGR), associated or not with preeclampsia, HELLP syndrome, gravidic steatosis, gravidic nephropathy or intra-uterine foetal death (IUFD), pregnancy diseases or infertility associated with auto immune pathologies in a subject comprising *in vitro* assaying for soluble MICA in a blood, plasma, or serum sample from a pregnant woman or a woman wishing to be pregnant, the level of soluble MICA being indicative of a probability of pregnancy complications and wherein the detection of soluble MICA in the serum sample of at least 0.3 ng/ml is indicative a higher probability of VPD, preeclampsia, vascular IUGR and/or IUFD.

9. A method for differentiating vascular from non vascular intra-uterine growth retardation (IUGR) in a subject comprising *in vitro* assaying for soluble MICA in a blood, plasma, or serum sample from a pregnant woman or a woman wishing to be pregnant, the level of soluble MICA being indicative of a probability of vascular IUGR, and wherein the sample is a serum sample and the detection of soluble MICA in the sample at at least 0.3 ng/ml is indicative a vascular IUGR.

10. Method according to any one of claims 8-9, wherein the level of soluble MICA is determined after embryo implantation.

11. The method according to anyone of claims 1-10, wherein the step of assaying for soluble MICA in the sample comprises contacting the sample with an anti-MICA antibody, preferably a monoclonal anti-MICA antibody, more preferably by ELISA assay, still more preferably by sandwich ELISA assay.

12. The method according to anyone of claims 1 to 11, wherein the assaying of soluble MICA is combined with the assaying of one or more additional markers preferably selected from the group consisting of HLA-G and angiogenic markers such as endoglin, PIGF and sFLT1.

13. Use of a kit comprising at least one element selected from the group consisting of an anti-MICA antibody, preferably a monoclonal anti-MICA antibody in the medicine and biology of the reproduction for determining in vitro fertilization (IVF) outcome according to any one of claims 1-7, for determining the probability of pregnancy complications according to claim 8 or for differentiating vascular from non vascular intra-uterine growth retardation (IUGR) in a subject according to any one of claims 9-10.

14. Use according to claim 13, wherein the kit further comprises:
- a soluble MICA protein; and/or
- one or more elements selected from the group consisting of antibody, primers and probes specific of one or more additional markers preferably selected from the group consisting of HLA-G and angiogenic markers such as endoglin, PIGF and sFLT1.

## Patentansprüche

1. Ein Verfahren zur Bestimmung des Ergebnisses einer *in vitro* Fertilisation (IVF) umfassend Untersuchen einer Blut-, Plasma- oder Serumprobe von einer Frau, die einen übertragenden Embryo erhält oder erhalten soll, *in vitro* auf lösliches MICA (MHC Klasse I Kettenbezogenes Protein A), die Menge an löslichem MICA ist ein Anzeichen für das IVF Ergebnis, insbesondere ein Anzeichen für Misserfolgsraten der Implantation, einen IVF Misserfolg und/oder eine Fehlgeburt.

2. Das Verfahren nach Anspruch 1, wobei eine geringe Menge an MICA ein Anzeichen für eine hohe Wahrscheinlichkeit für eine erfolgreiche IVF ist.

3. Das Verfahren nach Anspruch 1 oder 2, wobei die Menge an löslichem MICA vor Beginn einer hormonellen Konditionierungsbehandlung bestimmt wird und eine Menge an löslichem MICA in der Serumprobe kleiner als 2,45 ng/ml ein Anzeichen für eine hohe Wahrscheinlichkeit einer erfolgreichen IVF ist.

4. Verfahren nach Anspruch 1, wobei die Probe eine Serumprobe ist und das Subjekt die Frau ist, die den übertragenden Embryo erhalten soll, die Menge an löslichem MICA vor Beginn der hormonellen Konditionierungsbehandlung bestimmt wird und
- eine Menge an löslichem MICA größer als 2,45 ng/ml in der Serumprobe ein Anzeichen für hohe Misserfolgsraten der Implantation ist; und/oder
- eine Menge an löslichem MICA größer als 28 ng/ml in der Serumprobe ein Anzeichen für eine hohe Wahrscheinlichkeit für einen IVF Misserfolg ist; und/oder
- eine Menge an löslichem MICA größer als 6 ng/ml in der Serumprobe ein Anzeichen für eine hohe Wahrscheinlichkeit einer Fehlgeburt ist.

5. Verfahren nach Anspruch 1, wobei die Probe eine Serumprobe ist und das Subjekt eine Frau ist, die den übertragenden Embryo erhält, die Menge an löslichem MICA Protein auch nach der Implantation bestimmt werden kann und eine Menge an MICA größer als 3,2 ng/ml in der Serumprobe ein Anzeichen für eine hohe Wahrscheinlichkeit einer Fehlgeburt ist.

6. Verfahren nach Anspruch 1, wobei das Verfahren weiter das Identifizieren des Subjekts umfasst, das eine Menge an MICA aufweist, die ein Anzeichen für eine erfolgreiche IVF Wahrscheinlichkeit ist.

7. Verfahren nach Anspruch 6, wobei die Probe eine Serumprobe ist und die Menge an löslichem MICA vor Beginn der hormonellen Konditionierungsbehandlung bestimmt wird und eine lösliche MICA Menge in der Serumprobe kleiner als 28 ng/ml, vorzugsweise kleiner als 6 ng/ml und noch bevorzugter kleiner als 2,45 ng/ml ein Anzeichen für eine hohe Wahrscheinlichkeit einer erfolgreichen IVF ist.

8. Ein Verfahren zur Bestimmung der Wahrscheinlichkeit von Schwangerschaftskomplikationen umfassend Fehlgeburt, vaskuläre Schwangerschaftskrankheiten (VPD), Präeklampsie (PE), schwere Präeklampsie, vaskuläre intrauterine Wachstumsretardierung (IUGR), in Verbindung mit oder ohne Präeklampsie, HELLP Syndrom, in der Schwangerschaft auftretende Steatose, in der Schwangerschaft auftretende Nephropathie oder intrauteriner Tod des Fötus (IUFD), Schwangerschaftskrankheiten oder Unfruchtbarkeit in Verbindung mit autoimmun Pathologien in einem Subjekt umfassend Untersuchen einer Blut-, Plasma- oder Serumprobe von einer schwangeren Frau oder einer Frau, die sich wünscht, schwanger zu sein, *in vitro* auf lösliches MICA, die Menge an löslichem MICA ist ein Anzeichen für eine Wahrscheinlichkeit von Schwangerschaftskomplikationen und wobei der Nachweis von löslichem MICA in der Serumprobe von mindestens 0,3 ng/ml ein Anzeichen für eine hohe Wahrscheinlichkeit von VPD, Präeklampsie, vaskulärer IUGR und/oder IUFD ist.

9. Ein Verfahren zur Unterscheidung von vaskulärer von nicht vaskulärer intrauteriner Wachstumsretardierung (IUGR) in einem Subjekt umfassend Untersuchen einer Blut-, Plasma- oder Serumprobe von einer schwangeren Frau oder einer Frau, die sich wünscht, schwanger zu sein, *in vitro* auf lösliches MICA, die Menge an löslichem MICA ist ein Anzeichen für eine Wahrscheinlichkeit von vaskulärer IUGR, und wobei die Probe eine Serumprobe ist und der Nachweis an löslichem MICA in der Probe von mindestens 0,3 ng/ml ein Anzeichen für vaskuläre IUGR ist.

10. Verfahren nach einem der Ansprüche 8-9, wobei die Menge an löslichem MICA nach der Implantation des Embryos bestimmt wird.

11. Das Verfahren nach einem der Ansprüche 1-10, wobei der Schritt des Untersuchens auf lösliches MICA in der Probe das in Kontaktbringen der Probe mit einem anti-MICA Antikörper umfasst, vorzugsweise mit einem monoklonalen anti-MICA Antikörper, weiter bevorzugt durch einen ELISA Assay, noch weiter bevorzugt durch einen Sandwich-ELISA Assay.

12. Verfahren nach einem der Ansprüche 1 bis 11, wobei das Untersuchen auf lösliches MICA kombiniert wird mit der Untersuchung auf einen oder mehrere zusätzliche Marker vorzugsweise ausgewählt aus der Gruppe bestehend aus HLA-G und gefäßbildende Marker wie Endoglin, PIGF und sFLT1.

13. Verwendung eines Kits umfassend mindestens ein Element ausgewählt aus der Gruppe bestehend aus einem anti-MICA Antikörper, vorzugsweise einem monoklonalen anti-MICA Antikörper in der Medizin und der Reproduktionsbiologie zur Bestimmung des *in vitro* Fertilisation (IVF) Ergebnisses nach einem der Ansprüche 1-7, zur Bestimmung der Wahrscheinlichkeit von Schwangerschaftskomplikationen gemäß Anspruch 8 oder zur Unterscheidung vaskulärer von nicht vaskulärer intrauteriner Wachstumsretardierung (IUGR) in einem Subjekt nach einem der Ansprüche 9-10.

14. Verwendung nach Anspruch 13, wobei das Kit ferner umfasst:
- ein lösliches MICA Protein; und/oder
- ein oder mehrere Elemente ausgewählt aus der Gruppe bestehend aus Antikörper, Primern und Sonden spezifisch für ein oder mehrere zusätzliche Marker vorzugsweise ausgewählt aus der Gruppe bestehend aus HLA-G und gefäßbildenden Markern wie Endoglin, PIGF und sFLT1.

## Revendications

1. Méthode pour déterminer le résultat d'une fertilisation *in vitro* (FIV) comprenant l'analyse de MICA soluble (MHC class I chain-related protein A) dans un échantillon de sang, de plasma ou de sérum d'une femme recevant ou destinée à recevoir un embryon transféré, le niveau de MICA soluble étant indicatif du résultat de la FIV, en particulier des taux d'échec d'implantation, d'échec de FIV et/ou de fausse-couches.

2. Méthode selon la revendication 1, dans laquelle un faible niveau de MICA est indicatif d'une probabilité élevée de FIV réussie.

3. Méthode selon la revendication 1 ou 2, dans laquelle le niveau de MICA soluble est déterminé avant l'initiation du traitement hormonal de conditionnement et un niveau de MICA soluble dans un échantillon de sérum inférieur à 2,45 ng/ml est indicatif d'une probabilité élevée de FIV réussie.

4. Méthode selon la revendication 1, dans laquelle l'échantillon est un échantillon de sérum et le sujet est une femme qui est destinée à recevoir un embryon transféré, le niveau de MICA soluble est déterminé avant l'initiation du traitement hormonal de conditionnement et
- un niveau de MICA soluble supérieur à 2,45 ng/ml dans l'échantillon de sérum est indicatif de taux d'échec d'implantation plus élevés ; et/ou
- un niveau de MICA soluble supérieur à 28 ng/ml dans l'échantillon de sérum est indicatif d'une forte probabilité d'échec de FIV ; et/ou
- un niveau de MICA soluble supérieur à 6 ng/ml dans l' échantillon de sérum est indicatif d'une forte probabilité de fausse-couche.

5. Méthode selon la revendication 1, dans laquelle l'échantillon est un échantillon de sérum et le sujet est une femme qui a reçu l'embryon transféré, le niveau de protéine MICA soluble peut être aussi déterminé après l'implantation et un niveau de MICA supérieur à 3,2 ng/ml dans l'échantillon de sérum est indicatif d'une forte probabilité de fausse-couche.

6. Méthode selon la revendication 1, dans laquelle la méthode comprend en outre l'identification d'un sujet ayant un niveau de MICA indicatif d'une probabilité de FIV réussie.

7. Méthode selon la revendication 6, dans laquelle l'échantillon est un échantillon de sérum et le niveau de MICA soluble est déterminé avant l'initiation du traitement hormonal de conditionnement et un niveau de MICA soluble dans l'échantillon de sérum inférieur à 28 ng/ml, de préférence inférieur à 6 ng/ml, et de préférence encore inférieur à 2,45 ng/ml, est indicatif d'une forte probabilité de FIV réussie.

8. Méthode pour déterminer la probabilité de complications de grossesse, incluant une fausse-couche, des maladies vasculaires de grossesse (VPD), de pré-éclampsie (PE), de pré-éclampsie sévère, de retard de croissance intra-utérine vasculaire (IUGR), associé ou non avec une pré-éclampsie, un syndrome HELLP, un stéatose gravidique, une néphropathie gravidique ou une mort foetale intra-utérine (IUFD), des maladies de grossesse ou une infertilité associée à des pathologies auto-immunes, chez un sujet comprenant l'analyse *in vitro* de MICA soluble dans un échantillon de sang, de *plasma* ou de sérum d'une femme enceinte ou d'une femme désirant être enceinte, le niveau de MICA soluble étant indicatif d'une probabilité de complications de grossesse et dans laquelle la détection de MICA soluble dans l'échantillon de sérum d'au moins 0,3 ng/ml est indicative d'une probabilité plus élevée de VPD, pré-éclampsie, IUGR vasculaire et/ou IUFD.

9. Méthode pour différencier un retard de croissance intra-utérine (IUGR) vasculaire de non-vasculaire chez un sujet comprenant l'analyse *in vitro* de MICA soluble dans un échantillon de sang, de *plasma* ou de sérum d'une femme enceinte ou d'une femme désirant être enceinte, le niveau de MICA soluble étant indicatif d'une probabilité de IUGR vasculaire, et dans laquelle l'échantillon est un échantillon de sérum et la détection de MICA soluble dans l'échantillon d'au moins 0,3 ng/ml est indicative d'une IUGR vasculaire.

10. Méthode selon l'une quelconque des revendications 8-9, dans laquelle le niveau de MICA soluble est déterminé après l'implantation de l'embryon.

11. Méthode selon l'une quelconque des revendications 1-10, dans laquelle l'étape d'analyse de MICA soluble dans l'échantillon comprend la mise en contact de l'échantillon avec un anticorps anti-MICA, de préférence un anticorps anti-MICA monoclonal, de préférence encore par analyse ELISA, de manière encore plus préférée par analyse ELISA en sandwich.

12. Méthode selon l'une quelconque des revendications 1-11, dans laquelle l'analyse de MICA soluble est combinée avec l'analyse d'un ou plusieurs marqueurs supplémentaires, de préférence sélectionnés parmi le groupe consistant en HLA-G et des marqueurs angiogéniques tels que une endogline, PIGF et sFLT1.

13. Utilisation d'un kit comprenant au moins un élément sélectionné dans le groupe consistant en un anticorps anti-MICA, de préférence un anticorps anti-MICA monoclonal, en médecine et biologie de la reproduction pour déterminer le résultat d'une fertilisation *in vitro* (FIV) selon l'une quelconque des revendications 1-7, pour déterminer la probabilité de complications de grossesse selon la revendication 8 ou pour différencier un retard de croissance intra-utérine (IUGR) vasculaire de non-vasculaire chez un sujet selon l'une quelconque des revendications 9-10.

14. Utilisation selon la revendication 13, dans laquelle le kit comprend en outre :
- une protéine MICA soluble, et/ou
- un ou plusieurs éléments sélectionnés parmi le groupe consistant en un anticorps, des amorces et des sondes spécifiques d'un ou plusieurs marqueurs supplémentaires, de préférence sélectionnés parmi le groupe consistant en HLA-G et des marqueurs angiogéniques tels que une endogline, PIGF et sFLT1.


### SEQUENCE LISTING

<110> Université de la Méditérranée, et al.
<120> Biomarker for the medicine and the biology of the reproduction
<130> B0543WO
<160> 2
<170> PatentIn version 3.1
<210> 1
   <211> 22
   <212> DNA
   <213> artificial sequence
<220>
   <223> primer
<400> 1
   catgtttctg ctgttgctgc tg 22
<210> 2
   <211> 21
   <212> DNA
   <213> artificial sequence
<220>
   <223> primer
<400> 2
   gtggcatccc tgtggtcact c 21